# FASCICULE DE BREVET EUROPEEN

(11) **EP 0 291 436 B1**
(45) Date de publication et mention de la délivrance du brevet: **09.06.1993**
(21) Numéro de dépôt: 88450014.1
(22) Date de dépôt: 15.04.1988
(51) Int. Cl.: C07H 19/20

(54) **Procédé de production de l'adénosine-5' monophosphate d'heptaminol par atomisation d'une solution aqueuse le contenant**
Verfahren zur Herstellung von Heptaminoladenosin-5'-monophosphat durch Atomisierung einer wässerigen Lösung, welche dieses enthält
Process for the preparation of heptaminol adenosine-5'-monophosphate by atomisation of an aqueous solution containing it

(30) Priorité: 15.05.1987 PT 84874
(43) Date de publication de la demande: 17.11.1988
(73) Titulaire: LABORATOIRES SARGET S.A., 33701 Merignac (FR)
(72) Inventeur: Dumas, Jeanne, F-33200 Bordeaux (FR); Feniou, Claude, F-33600 Pessac (FR)
(74) Mandataire: Phélip, Bruno

(56) Documents cités:
- DE-A- 3 437 832
- FR-A- 2 260 331

## Description

Cette invention est relative à un procédé de production de l'Adénosine-5′ monophosphate d'heptaminol.

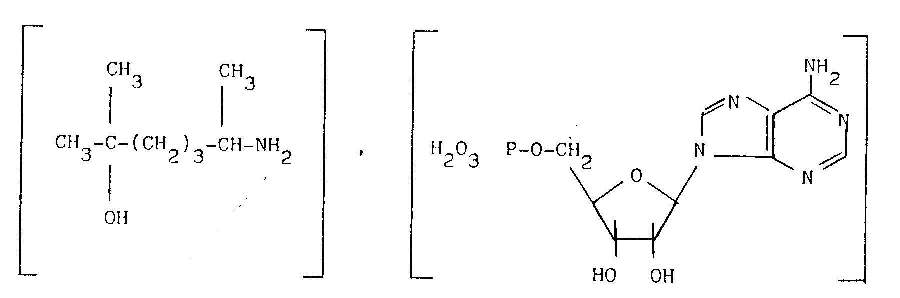

Ce composé tout particulièrement utile pour le traitement des insuffisances vasculaires veineuses, peut être obtenu selon un ancien procédé (brevet français numéro 74 04 237 du Centre Européen de Recherches Mauvernay). Ce procédé est caractérisé par le fait que l'on procède à la lyophilisation d'une solution aqueuse contenant en quantité équimoléculaire de l'heptaminol base et de l'acide Adénosine-5′ monophosphorique, pour obtenir le sel sous forme de poudre blanche. Cette méthode présente les inconvénients d'être longue et coûteuse.

Nous avons découvert que la technique d'atomisation pouvait parfaitement s'appliquer à la production de l'Adénosine-5′ monophosphate d'hepataminol. Ce nouveau procédé rapide et bon marché produit ce sel sous forme de poudre blanche répondant à toutes les caractéristiques physicochimiques qui lui sont propres.

Cette invention est décrite plus précisemment dans l'exemple suivant :
Dans un réacteur de 4000 litres on introduit successivement à température ambiante et sous agitation :
- 2 250 1 d'eau déminéralisée
- 225 kg d'heptaminol base
- 539 kg d'acide adénosine-5′ monophosphorique

Au bout de quelques minutes la dissolution est complète.

A ce moment, la solution obtenue est introduite en tête d'un atomiseur à bol au débit de 320 litres à l'heure. Les températures d'entrée et de sortie de l'atomiseur sont fixées respectivement à 160°C et 90°C.

L'atomisation est immédiate et la poudre produite est récupérée dans un filtre à éanches puis conditionnée en fûts étanches de 100 litres.

Des variantes opératoires concernant la concentration (25%) de la solution à atomiser et les températures d'entrée (entre 150°C et 190°C) et sortie (entre 70°C et 100°C) de l'atomiseur sont possibles fournissant l'Adénosine-5′ monophosphate d'heptaminol parfaitement conforme aux spécifications.

## Revendications

1. Procédé de production de l'Adénosine-5' monophosphate d'heptaminol par atomisation d'une solution aqueuse le contenant.

2. Procédé selon la revendication 1, caractérisé en ce que la concentration de l'Adénosine-5' monophosphate d'heptaminol en solution aqueuse est de 25 %.

3. Procédé selon la revendication 1, caractérisé en ce que la température d'entrée en tête de l'atomiseur peut prendre toute valeur entre 150°C et et 190°C.

4. Procédé selon la revendication 1, caractérisé en ce que la température de sortie de l'atomisateur peut prendre toute valeur entre 70°C et 100°C.

5. Procédé selon la revendication 1, caractérisé en ce que la poudre formée est récupérée par filtration sur filtre à manches.

## Claims

1. Process for producing heptaminol adenosine-5' monophosphate by spraying an aqueous solution containing it.

2. Process according to claim 1 , characterized in that the heptaminol adenosine-5' monophosphate concentration in the aqueous solution is 25 %.

3. Process according to claim 1 , characterized in that the inlet temperature in the head of the atomizer can be comprised between 150 °C and 190°C.

4. Process according to claim 1, characterized in that the outlet temperature in the atomizer can be comprised between 70°C and 100°C.

5. Process according to claim 1, characterized in that the formed powder is collected by filtration on a bag filter.

## Patentansprüche

1. Verfahren zur Herstellung von Heptaminoladenosin-5'-monophosphat durch Atomisierung einer wäßrigen Lösung, welche dieses enthält.

2. Verfahren nach Anspruch 1, dadurch gekennzeichnet, daß die Konzentration von Heptaminoladenosin-5'-monophosphat in der wäßrigen Lösung 25% ist.

3. Verfahren nach Anspruch 1, dadurch gekennzeichnet, daß die Eintrittstemperatur im Kopf des Zerstäubers jeden Wert zwischen 150 °C und 190 °C annehmen kann.

4. Verfahren nach Anspruch 1, dadurch gekennzeichnet, daß die Austrittstemperatur des Zerstäubers jeden Wert zwischen 70 °C und 100 °C annehmen kann.

5. Verfahren nach Anspruch 1, dadurch gekennzeichnet, daß das gebildete Pulver durch Filtration über ein Beutelfilter wiedergewonnen wird.
